**Europäisches Patentamt**

**European Patent Office**

**Office européen des brevets**

(11) Veröffentlichungsnummer: **0 185 622 B1**

(12) **EUROPÄISCHE PATENTSCHRIFT**

(45) Veröffentlichungstag der Patentschrift:
**27.09.89**

(51) Int. Cl.⁴: **C 07 D 301/32, C 07 D 303/27, C 08 G 59/00, C 08 G 59/08**

(21) Anmeldenummer: **85810602.4**

(22) Anmeldetag: **16.12.85**

(54) Verfahren zur Verminderung des Gehaltes an hydrolysierbarem Chlor in Glycidylverbindungen.

(30) Priorität: **21.12.84 CH 6098/84**

(43) Veröffentlichungstag der Anmeldung:
**25.06.86 Patentblatt 86/26**

(45) Bekanntmachung des Hinweises auf die Patenterteilung:
**27.09.89 Patentblatt 89/39**

(84) Benannte Vertragsstaaten:
**CH DE FR GB IT LI NL**

(56) Entgegenhaltungen:
**GB-A- 1 386 594**
**US-A- 4 485 221**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Darbellay, Jacques, Rue des Raffineries, CH-1893 Muraz-Collombey (CH)**
Erfinder: **Dessauges, Gerald, Dr., Ruelle du Chêne 1, CH-1820 Montreux (CH)**

## Beschreibung

Die vorliegende Erfindung betrifft ein Verfahren zur Verminderung des Gehaltes an hydrolysierbarem Chlor in Glycidylverbindungen durch deren Nachbehandlung mit überschüssigem Alkalimetallhydroxid und anschließende Behandlung mit feuchter Cellulose.

Bekanntlich sind die mittels Epichlorhydrin hergestellten Glycidylverbindungen, insbesondere die technisch hergestellten, stets mit Chlor verunreinigt, das im Epoxidharz als ionisches Chlor und in der Glycidylverbindung als hydrolysierbares (1,2-Chlorhydrin) und als nichthydrolysierbares Chlor (Chlormethyl) vorliegt.

An Epoxidharze, insbesondere solche, die zur Herstellung von elektrischen und elektronischen Bauteilen Verwendung finden, werden laufend höhere Ansprüche bezüglich Reinheit gestellt, um den Korrosionseinfluß des Restchlorgehaltes auf Substrate, insbesondere Kontaktmetalle, zu mindern.

Es sind bereits viele Methoden zur Entfernung des Restchlorgehaltes aus Epoxidharzen bekannt geworden. Wie im «Handbook of Epoxy Resins» (1967), 4–30, von H. Lee und K. Neville dargelegt wird, sind diese Methoden auch mit Nachteilen verbunden.

Sowohl im GB-Patent 1 278 737 als auch in der DE-Offenlegungsschrift 25 23 696 werden spezifische Verfahren zur Herstellung von Polyglycidyläthern durch zweimalige Dehydrohalogenisierung mittels Natronlauge offenbart. Der Anteil an Gesamtchlor in den verfahrengemäß erhaltenen Polyglycidyläthern ist noch relativ hoch. In der JP-Patentanmeldung Kokai 58-173116 wird vorgeschlagen, den Restchlorgehalt in Epoxidharzen mittels Silbersalzen organischer Säuren zu entfernen. Abgesehen davon, daß es sich hierbei um ein kostspieliges Verfahren handelt, haben eigene Nacharbeitungen ergeben, daß der nach diesem Verfahren erzielte Effekt gering ist.

Es wurde nun gefunden, daß man den Gehalt an hydrolysierbarem Chlor in Glycidylverbindungen sehr verringern kann, wenn man diese in einem organischen Lösungsmittel bei erhöhter Temperatur mit einem Überschuß an Alkalimetallhydroxiden und danach bei erhöhter Temperatur mit feuchter Cellulose behandelt, wobei das bei der Dehydrochlorierung entstehende ionische Chlor ebenfalls weitgehend aus der Epoxidharzmasse entfernt wird. Bei dem erfindungsgemäßen Verfahren ist es nicht erforderlich, nach der Alkalimetallhydroxidbehandlung das überschüssige Alkalimetallhydroxid in der Reaktionslösung zu neutralisieren oder aus ihr vor der weiteren Behandlung mit feuchter Cellulose zu entfernen.

Gegenstand vorliegender Erfindung ist somit ein Verfahren zur Verminderung des Gehaltes an hydrolysierbarem Chlor in Glycidylverbindungen durch Nachbehandlung der Glycidylverbindungen mit Alkalimetallhydroxiden, das dadurch gekennzeichnet ist, daß man die in einem halogenfreien organischen Lösungsmittel gelöste Glycidylverbindung bei erhöhter Temperatur mit einer wäßrigen Alkalimetallhydroxidlösung im äquivalenten Überschuß behandelt, wobei der Überschuß 100 bis 200% der zur vollständigen Dehydrochlorierung des hydrolysierbaren Chlors theoretisch erforderlichen äquivalenten Menge beträgt, das Wasser aus der organischen Lösung entfernt, bei erhöhter Temperatur die organische Lösung mit der 5- bis 500fachen Menge feuchter, bis zu 35 Gew.-% Wasser enthaltender Cellulose, bezogen auf die eingesetzte Menge Alkalimetallhydroxid, behandelt und nach dem Filtrieren die Glycidylverbindung aus der organischen Lösung isoliert.

Für das erfindungsgemäße Verfahren können Glycidylverbindungen eingesetzt werden, die direkt an Sauerstoff-, Stickstoff- oder Schwefelatome gebundene Glycidylgruppen enthalten. Als Beispiele solcher Harze seien Polyglycidyl- und Poly(ß-methyl-glycidyl)-ester genannt, die man durch Umsetzung einer zwei oder mehr Carbonsäuregruppen pro Molekül enthaltenden Verbindung mit Epichlorhydrin, Glycerindichlorhydrin oder ß-Methylepichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Pimelinsäure, Korksäure, Azelainsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, und von aromatischen Polycarbonsäuren, wie Phthalsäure, Isophthalsäure und Terephthalsäure, ableiten.

Weitere Beispiele sind Polyglycidyl- und Poly(ß-methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Äther lassen sich mit den genannten Glycidylierungsmitteln beispielsweise aus acyclischen Alkoholen, wie Äthylenglykol, Diäthylenglykol, höhere Poly-(oxyäthylen)-glykole, Propan-1,2-diol, Poly(oxy-propylen)-glykole, Propan-1,3-diol, Poly-(oxytetramethylen)-glykole, Pentan-1,5-diol, Hexan-2,4,6-triol, Glycerin,1,1,1-Trimethylolpropan, Pentaerythrit und Sorbit. aus cycloaliphatischen Alkoholen, wie Resorcit, Chinit, Bis-(4-hydroxycyclohexyl)-methan, 2,2-Bis-(4-hydroxycyclohexyl)-propan und 1,1-Bis-(hydroxymethyl)-cyclohexan, und aus Alkoholen mit aromatischen Kernen, wie N,N-Bis-(2-hydroxyäthyl)-anilin und p,p'-Bis-(2-hydroxyäthyl)-anilin und p,p'-Bis-(2-hydroxyäthylamino)-diphenylmethan, herstellen. Man kann sie ferner aus einkernigen Phenolen, wie Resorcin und Hydrochinon, und mehrkernigen Phenolen, wie Bis-(4-hydroxyphenyl)-methan Bisphenol F 4,4'-Dihydroxydiphenyl, 1,1,2,2-Tetrakis-(4-hydroxyphenyl)-methan, 2,2-Bis(4-hydroxyphenyl)-propan Bisphenol A und aus durch Umsetzung von Aldehyden, wie Formaldehyd, Acetaldehyd und Benzaldehyd, mit Phenol oder alkylsubstituiertem Phenol, wobei die Alkylgruppen jeweils bis zu neun Kohlenstoffatomen enthalten können. wie 2-Methylphenol und 4-tert.-Buthylphenol, gebildeten Novolaken erhalten.

Poly-(n-glycidyl)-verbindungen sind ebenfalls für das erfindungsgemäße Verfahren verwendbar. z.B N-Glycidylderivate von Aminen, wie Anilin, n-Butylamin, Bis-(4-aminophenyl)-methan und Bis-(4-methylaminophenyl)-methan, Triglycidylisocyanurat sowie N,N'-Diglycidylderivate von cyclischen Alkylenharnstoffen, wie Äthylenharnstoff und 1,3-Propylenharnstoff, und von Hydantoinen, wie 5,5-Dimethylhydantoin.

Auch kann man Poly-(S-glycidyl)-verbindungen einsetzen, z.B. Di-(S-glycidyl)-derivate von Dithiolen, wie Äthan-1,2-dithiol und Bis-(4-mercaptomethylphenyl)-äther, doch sind diese nicht bevorzugt.

In Betracht kommen auch Glycidylverbindungen, in welchen die Glycidylgruppen an verschiedene Heteroatome gebunden sind, beispielsweise p-(Diglycidylamino)-phenylglycidyläther.

Vorzugsweise setzt man beim erfindungsgemäßen Verfahren die Glycidyläther, insbesondere die der mehrkernigen Phenole, ein.

Geeignete halogenfreie organische Lösungsmittel, die beim erfindungsgemäßen Verfahren eingesetzt werden können, sind beispielsweise aliphatische Kohlenwasserstoffe, wie Pentan, Hexan, Heptan oder Oktan, cycloaliphatische Kohlenwasserstoffe, wie Cyclohexan oder Cyclopentan, aromatische Kohlenwasserstoffe, wie Benzol, Tuluol oder Xylole, aliphatische oder cyclische Äther, wie Diäthyläther, Dioxan oder Tetrahydrofuran und Ketone, wie Methyläthylketon oder Methylisobutylketon.

Zur Behandlung der Glycidylverbindungen mit wäßrigen Alkalimetallhydroxiden verwendet man vorzugsweise Natronlauge. Die Behandlung erfolgt vorzugsweise im Temperaturbereich von 60 bis 130°C, indem man zu der in einem organischen Lösungsmittel gelösten Glycidylverbindung unter Rühren die wäßrige Alkalimetallhydroxidlösung nach und nach zugibt. Dabei setzt man im allgemeinen 20- bis 90 gew.-%ige wäßrige Alkalimetallhydroxidlösungen ein, wobei diese zur Behandlung von Glycidyläthern vorzugsweise in solchen Mengen zugegeben werden, daß der Überschuß 100 bis 170%, insbesondere 120 bis 170%, der theoretisch erforderlichen äquivalenten Menge beträgt.

Bei der Behandlung von N-Glycidylverbindungen beträgt der Überschuß vorzugsweise 150–200%.

Je nach angewendeter Temperatur und Konzentration der eingesetzten Alkalimetallhydroxidlösung dauert die Behandlung zwischen etwa 10 Minuten und etwa einer Stunde. Beispielsweise genügt bei Verwendung einer 30-gew.-%igen Natronlauge die Behandlungsdauer von etwa einer halben Stunde, wenn, diese bei etwa 80°C vorgenommen wird. Nach dieser Behandlung wird das Wasser aus der organischen Lösung entfernt, vorzugsweise mittels azeotroper Destillation.

Die Behandlungsdauer der die Glycidylverbindung enthaltenden organischen Lösung mit feuchter, bis zu 35 Gew.-% Wasser enthaltender Cellulose, bezogen auf das Trockengewicht der Cellulose, kann innerhalb weiter Grenzen variieren, beträgt im Allgemeinen mindestens 20 Minuten. vorzugsweise ½ bis 1 Stunde. Die Behandlung mit Cellulose wird vorzugsweise im Temperaturbereich von 30 bis 90°C insbesondere zwischen 50 und 80°C, vorgenommen und erfolgt vorzugsweise unter ständigem Rühren der organischen Lösung.

Vorzugsweise enthält die eingesetzte Cellulose bis zu 30 Gew.-% Wasser und mindestens etwa 5 Gew.-%, insbesondere mindestens etwa 10 Gew.-% Wasser.

Die verwendete feuchte Cellulose wird durch einfaches Anfeuchten von im Handel erhältlicher Cellulose mit der entsprechenden Menge Wasser hergestellt. Als Cellulose können sowohl die faserförmige Cellulose, erhältlich im Handel beispielsweise unter der Bezeichnung Solka Floc von der Firma Brown, USA, als auch die pulverförmige Cellulose, wie sie im Handel beispielsweise unter der Bezeichnung Arbocel® von der Firma Rettenmaier & Söhne, DE, erhältlich ist, eingesetzt werden.

Nach dem Abfiltrieren der Cellulose werden die Glycidylverbindungen durch Entfernen des organischen Lösungsmittels erhalten. Wie bereits erwähnt, sind die nach dem erfindungsgemäßen Verfahren gereinigten Glycidylverbindungen besonders für Anwendungen auf dem Elektroniksektor geeignet.

Die in den folgenden Beispielen angegebenen Mengen an hydrolysierbarem Chlor und ionischem Chlor werden potentiometrisch nach folgender Methode bestimmt:

10 g der Glycidylverbindung werden in 100 ml Aceton gelöst. Dann werden 1 ml 0,001 n wäßrige NaCl-Lösung, 1 ml Essigsäure und 2 ml demineralisiertes Wasser zugegeben. Die potentiometrische Bestimmung des Chlorgehaltes erfolgt unverzüglich mittels einer 0,001 n Silbernitratlösung in Essigsäure.

Die Cl-Bestimmungsmethode wird als Blindversuch – ohne Glycidylverbindung – wiederholt. Aus dem Differenzbetrag läßt sich der reale Chlorgehalt ermitteln.

Beispiel 1:

400 g eines technisch aus Kresolnovolak und Epichlorhydrin in Gegenwart von Natronlauge hergestellten Kresolnovolak-Epoxidharzes mit einem Epoxidgehalt von 4,62 Äquivalenten/kg, einem Gehalt an hydrolysierbarem Chlor von 0,095 Gew.-%, und einer Viskosität von 2750 mPa·s bei 130 °C werden bei Raumtemperatur in Methylisobutylketon (MIK) vollständig gelöst und auf 85 °C erhitzt. Dann werden 1,9 g einer 29,8 gew.-%igen wäßrigen Natronlauge innerhalb von 30 Minuten unter Rühren zugegeben. Die Lösung wird auf 113 °C erhitzt, wobei Wasser aus der Lösung azeotrop entfernt wird bis der Wassergehalt weniger als 0,1 Gew.-% beträgt. Nach dem Kühlen auf 60°C werden 4,6 g Wasser zugegeben und die Lösung während 10 Minuten gerührt. Dann werden 35 g feuchte Cellulose (Arbocel® 874) mit einem Wassergehalt von 30 Gew.-% zur Lösung gegeben und diese 30 Minuten lang gerührt. Anschließend wird die Lösung unter Verwendung eines 100 Mikronfilters filtriert. Aus dem Filtrat wird das Lösungsmittel mittels Vakuumdestillation, wobei das Vakuum allmählich auf 135°C/30 mbar gesteigert wird. entfernt. Dann wird das Harz zwecks Entfernung aller flüchtigen organischen Bestandteile mit Wasser und danach mit Stickstoffgas gestrippt. Man erhält einen Kresolnovolak-Epoxidgehalt von 4,62 Äquivalenten kg, einen Gehalt an hydrolisierbarem Chlor von 0,0028 Gew.-%, einen Gehalt an ionischem Chlor von weniger als 0,0001 Gew.-% und eine Viskosität von 3060 mPa·s bei 130°C.

**Beispiel 2:**

Nach der gleichen in Beispiel 1 angegebenen Arbeitsweise werden 100 g eines technisch aus Tetra-(p-hydrophenyl)-äthan und Epichlorhydrin in Gegenwart von Natronlauge hergestellten Tetra-(p-glycidyl-oxyphenyl)-äthans mit einem Epoxidgehalt von 4,78 Äquivalenten/kg und einem Gehalt an hydrolysierbarem Chlor von 0,18 Gew.-% in MIK gelöst und mit 1,0 g einer 30 gew.-%igen wäßrigen Natronlauge und mit 16 g feuchter Cellulose, enthaltend 30 Gew.-% Wasser, behandelt. Man erhält Tetra(p-glycidyloxyphenyl)-äthan mit einem Epoxydgehalt von 4,80 Äquivalenten/kg, einem Gehalt an hydrolysierbarem Chlor von 0,0063 Gew.-% und einem Gehalt an ionischem Chlor von kleiner als 0,0001 Gew.-%.

**Beispiel 3:**

Nach der in Beispiel 1 angegebenen Arbeitsweise werden 100 g eines aus Bisphenol A und Epichlorhydrin in Gegenwart von Natronlauge technisch hergestellten Bisphenol A-diglycidyläthers mit Epoxidgehalt von 5,24 Äquivalenten/kg, einem Gehalt an hydrolysierbarem Chlor von 0,30 Gew.-% und einer Viskosität von 13 400 mPa·s bei 25 °C im MIK gelöst und mit 1,3 g einer 30 gew.-%igen wäßrigen Natronlauge und mit 20 g feuchter Cellulose, enthaltend 30 Gew.-% Wasser, behandelt. Die Harzlösung wird durch ein 10-Mikronfilter filtriert und wie in Beispiel 1 aufgearbeitet.

Man erhält Bisphenol A-diglycidyläther mit einem Epoxidgehalt von 5,28 Äquivalenten/kg, einem Gehalt an hydrolisierbarem Chlor von 0,0011 Gew.-% und einem Gehalt an ionischem Chlor von kleiner als 0,0001 Gew.-%.

**Beispiel 4:**

400 g des in Beispiel 1 verwendeten Kresolnovolak-Epoxidharzes werden in 600 g Toluol gelöst und die Lösung wird auf 85°C erhitzt. Zu dieser Lösung werden 2,41 g einer 30,24 gew.-%igen wäßrigen Natronlauge innerhalb von 15 Minuten unter Rühren zugegeben. Dann wird die Lösung auf 112 °C erhitzt, wobei solange Wasser azeotrop aus der Lösung entfernt wird bis diese einen Wassergehalt von kleiner als 0,03 Gew.-% aufweist. Nach dem Kühlen auf 65 °C werden zur Lösung 5,84 g Wasser zugegeben, und die Lösung wird 5 Minuten gerührt. Dann werden 34,7 g feuchte, 1 Gew.-% Wasser enthaltende Cellulose zur Lösung zugegeben und diese während 15 Minuten gerührt. Anschließend wird die Lösung unter Verwendung eines 100-Mikronfilters filtriert. Aus dem Filtrat wird das Lösungsmittel mittels Vakuumdestillation entfernt, wobei die Temperatur allmählich auf 135 °C gebracht wird und das Vakuum bis zu 220 mbar erniedrigt wird. Dann wird das Harz zwecks Entfernung aller flüchtigen organischen Bestandteile mit Wasser und anschließend mit Stickstoffgas gestrippt. Man erhält ein Kresolnovolak-Epoxidharz mit einem Epoxidgehalt von 4,65 Äquivalenten/kg, einem Gehalt an hydrolisierbarem Chlor von 0,0035 Gew.-%, einem Gehalt an ionischem Chlor von 0,0001 Gew.-% und einer Viskosität von 3450 mPa·s bei 130 °C.

**Patentansprüche**

1. Verfahren zur Verminderung des Gehaltes an hydrolysierbarem Chlor in Glycidylverbindungen durch Nachbehandlung der Glycidylverbindungen mit Alkalimetallhydroxiden, dadurch gekennzeichnet, daß man die in einem halogenfreien organischen Lösungsmittel gelöste Glycidylverbindung bei erhöhter Temperatur mit einer wäßrigen Alkalimetallhydroxidlösung im äquivalenten Überschuß behandelt, wobei der Überschuß 100 bis 200% der zur vollständigen Dehydrochlorierung des hydrolysierbaren Chlors theoretisch erforderlichen äquivalenten Menge beträgt das Wasser aus der organischen Lösung entfernt, bei erhöhter Temperatur die organische Lösung mit der 5- bis 500fachen Menge feuchter, bis zu 35 Gew.-% Wasser enthaltender Cellulose, bezogen auf die eingesetzte Menge Alkalimetallhydroxid, behandelt und nach dem Filtrieren die Glycidylverbindung aus der organischen Lösung isoliert.

2. Verfahren gemäß Anspruch 1, dadurch gekennzeichnet, daß man als Glycidylverbindung einen Glycidyläther einsetzt.

3. Verfahren gemäß Anspruch 1, dadurch gekennzeichnet, daß man als Glycidylverbindung einen Glycidyläther eines mehrkernigen Phenols einsetzt.

4. Verfahren gemäß Anspruch 1, dadurch gekennzeichnet, daß man die Behandlung der Glycidylverbindung mit Alkalimetallhydroxid im Temperaturbereich von 60 bis 130 °C vornimmt.

5. Verfahren gemäß Anspruch 3, dadurch gekennzeichnet, daß der Überschuß an Alkalimetallhydroxid 100 bis 170%, vorzugsweise 120 bis 170% der theoretisch erforderlichen äquivalenten Menge beträgt.

6. Verfahren gemäß Anspruch 1, dadurch gekennzeichnet, daß die feuchte Cellulose bis zu 30 Gew.-% Wasser, bezogen auf das Trockengewicht der Cellulose, enthält.

7. Verfahren gemäß Anspruch 1, dadurch gekennzeichnet, daß man die Behandlung der organischen Lösung mit Cellulose im Temperaturbereich von 30 bis 90 °C vornimmt.

8. Verfahren gemäß Anspruch 1, dadurch gekennzeichnet, daß man die Behandlung der organischen Lösung mit Cellulose im Temperaturbereich von 50 bis 80 °C vornimmt.

9. Verfahren gemäß Anspruch 1, dadurch gekennzeichnet, daß man eine 10- bis 100fache Menge Cellulose einsetzt.

10. Verfahren gemäß Anspruch 1, dadurch gekennzeichnet, daß man pulverförmige Cellulose einsetzt.

**Claims**

1. A process for reducing the content of hydrolysable chlorine in a glycidyl compound by after-treatment of the glycidyl compound with an alkali metal hydroxide, which comprises treating the glycidyl compound, dissolved in a halogen-free organic solvent, with an equivalent excess of an aqueous alkali

metal hydroxide solution at elevated temperature, the excess being 100 to 200% of the equivalent amount theoretically required for complete dehydrochlorination of the hydrolysable chlorine, removing the water from the organic solution, treating the organic solution with 5 to 500 times the amount, based on the amount of alkali metal hydroxide employed, of moist cellulose containing up to 35% by weight of water at elevated temperature and, after filtration, isolating the glycidyl compound from the organic solution.

2. The process according to claim 1, wherein a glycidyl ether is employed as the glycidyl compound.

3. The process according to claim 1, wherein a glycidyl ether of a polynuclear phenol is employed as the glycidyl compound.

4. The process according to claim 1, wherein the treatement of the glycidyl compound with alkali metal hydroxide is carried out in the temperature range from 60 to 130 °C.

5. The process according to claim 3, wherein the excess of alkali metal hydroxide is 100 to 170%, preferably 120 to 170%, of the theoretically required equivalent amount.

6. The process according to claim 1, wherein the moist cellulose contains up to 30% by weight of water, based on the dry weight of the cellulose.

7. The process according to claim 1, wherein the treatment of the organic solution with cellulose is carried out in the temperature range from 30 to 90 °C.

8. The process according to claim 1, wherein the treatment of the organic solution with cellulose is carried out in the temperature range from 50 to 80 °C.

9. The process according to claim 1, wherein 10 to 100 times the amount of cellulose is employed.

10. The process according to claim 1, wherein pulverulent cellulose is employed.

**Revendications**

1. Procédé pour abaisser la teneur en chlore hydrolysable dans des composés glycidyliques selon lequel on soumet les composés glycidyliques à un traitement complémentaire par des hydroxydes de métaux alcalins, procédé caractérisé en ce qu'on traite le composé glycidylique dissous dans un solvant organique non halogéné, à température élevée, par un excès, en équivalents, d'une solution aqueuse d'un hydroxyde de métal alcalin, l'excès représentant de 100 à 200% de la quantité équivalente qui est théoriquement nécessaire pour déshydrochlorer complètement le chlore hydrolysable, on élimine l'eau de la solution organique, on traite la solution organique, à température élevée, par une quantité de cellulose humide, contenant au plus 35% en poids d'eau, qui représente de 5 à 500 fois la quantité d'hydroxyde de métal alcalin mise en jeu, et, après filtration, on isole le composé glycidylique de la solution organique.

2. Procédé selon la revendication 1 caractérisé en ce qu'on utilise, comme composé glycidylique, un éther glycidylique.

3. Procédé selon la revendication 1 caractérisé en ce qu'on utilise, comme composé glycidylique, un éther glycidylique d'un phénol à plusieurs noyaux.

4. Procédé selon la revendication 1 caractérisé en ce que le traitement du composé glycidylique par l'hydroxyde de métal alcalin est effectué dans l'intervalle de température allant de 60 à 130 °C.

5. Procédé selon la revendication 3 caractérisé en ce que l'excès de l'hydroxyde de métal alcalin est de 100 à 170%, de préférence de 120 à 170%, de la quantité équivalente théoriquement nécessaire.

6. Procédé selon la revendication 1 caractérisé en ce que la cellulose humide contient jusqu'à 30% en poids d'eau par rapport au poids sec de la cellulose.

7. Procédé selon la revendication 1 caractérisé en ce que le traitement de la solution organique par la cellulose est exécuté dans l'intervalle de température allant de 30 à 90°C.

8. Procédé selon la revendication 1 caractérisé en ce que le traitement de la solution organique par la cellulose est exécuté dans l'intervalle de température allant de 50 à 80 °C.

9. Procédé selon la revendication 1 caractérisé en ce qu'on utilise une quantité cellulose de 10 à 100 fois.

10. Procédé selon la revendication 1 caractérisé en ce qu'on utilise de la cellulose pulvérulente.